# EUROPEAN PATENT SPECIFICATION

(11) **EP 1 028 671 B1**
(45) Date of publication and mention of the grant of the patent: **12.01.2005**
(21) Application number: 98956574.2
(22) Date of filing: 03.11.1998
(51) Int. Cl.: A61F 2/01, A61M 25/01

(54) **TEMPORARY VASCULAR FILTER GUIDE WIRE**
FÜHRUNGSDRAHT FÜR TEMPORÄREN BLUTFILTER
FIL-GUIDE TEMPORAIRE D'UN FILTRE VASCULAIRE

(30) Priority: 03.11.1997 US 963524
(43) Date of publication of application: 23.08.2000
(73) Proprietor: C.R. Bard Inc., Murray Hill, NJ 07974 (US)
(72) Inventor: GRAY, William, Mercer Island, WA 98040 (US); GAMBALE, Richard, A., Tyngsboro, MA 01879 (US)
(74) Representative: HOFFMANN - EITLE
(86) International application number: PCT/US1998/023516
(87) International publication number: WO 1999/022673

(56) References cited:
- EP-A- 0 533 511
- WO-A-95/34254
- WO-A-96/01591
- FR-A- 2 567 405
- US-A- 4 723 549
- US-A- 4 794 928
- US-A- 4 926 858
- US-A- 5 108 419

## Description

### Field of the Invention

The invention relates to vascular filters intended to capture embolic particles, by means of filtration, that may arise from the treatment of diseased blood vessels. A vascular filter guidewire according to the preamble of claim 1 is disclosed in EP-A-0 533 511. In addition WO-A-96/01591 discloses a method of fabricating a vascular filter.

### Background of the Invention

Percutaneous intravascular treatment of diseased blood vessels, such as angioplasty or stent placement procedures, may result in the dislodgment of loose plaque or thrombus which then migrate downstream. Since any such particles may become lodged in other vessels, effectively preventing blood from passing into the organ which that vessel supplies, and potentially causing serious end-organ damage which may be difficult or impossible to reverse, effective avoidance of this complication is extremely important.

One of the early methods of removing residual matter resulting from an angioplasty procedure using a balloon catheter involved maintaining the balloon in an inflated state while performing the intended intervention on the blood vessel. In this manner, much of the material could be removed without an extraneous filtering device. However, the reliability of such a procedure, especially for blood vessels supplying oxygen to the brain, necessitated substantial improvement.

Previous attempts at vascular filters have included a vena caval filter, which is permanently deployed in the vena cava via a peripheral vein in order to prevent embolisation of blood clots from the veins of the legs to the lungs, thus avoiding potentially serious and life threatening pulmonary embolism. The filter typically included a plurality of anchoring legs bent outwardly to form hooks to penetrate the vessel wall and secure the filter permanently in position. An example of such a device is disclosed in U.S. Patent No. 4,619,246.

While conventional vena caval filters work well for their intended purposes, they suffer from the disadvantages associated with damaging the inner vessel wall through the inherent penetrating nature of the hooks, and blockage caused over time as the filter becomes endothelialized with the blood vessel inner wall or as recurrent blood clots obstruct blood flow through the filter.

In an effort to resolve the problems with vena caval filters, those skilled in the art have developed temporary filtering mechanisms that attach to an angioplasty catheter and withdraw from the vasculature following the procedure. One proposal, disclosed in U.S. Patent No. 4, 723, 549, discloses a collapsible wire mesh filter disposed around the distal portion of a wire guided balloon catheter. A filter balloon is positioned beneath the wire mesh and inflates radially outwardly to expand the wire mesh when inserted downstream of a stenosed blood vessel. As the vessel is treated, fine particles dislodged from the stenosis are trapped by the mesh and subsequently removed with the filter and catheter following the procedure.

A similar device and method, disclosed in U.S. Patent No. 4, 873, 978 includes a balloon catheter directed through a vasculature by a guide wire. The catheter mounts a strainer at its distal end that responds to actuation of a separate control wire to open and close a plurality of tines capable of retaining dislodged particles from a treated stenosis.

The temporary filter devices described above require additional lumens and/or control wires beyond those associated with the catheter guide wire to control the filtering procedure. The extra lines and wires typically create added complexity for the operator. Moreover, it is often desirable to adjust the relative spacing between the deployed filter and the stenosed area due to the potential presence of additional blood vessels proximate the stenosis. Because the conventional filters are mounted to the distal ends of the respective catheters, adjustments during the procedure typically cannot be made. Furthermore, the use of balloon catheters and stent devices involving the same procedure could not be achieved with filter protection in place. An atherectomy device for severe occlusions, having a retention member in form of an expandable braided basket which is carried by a guide wire is disclosed in U.S. Patent No. 4,926,858.

Still, the need exists in the art for a temporary vascular filter which permits adjustment of the filter with respect to a lesioned vessel area, and allows for the exchange of various types of devices (e.g., balloon catheters, stents, etc.), while maintaining protection against distal emboli. The temporary vascular filter guide wire of the present invention satisfies these needs.

### SUMMARY OF THE INVENTION

Aspects of the present invention are defined in claims 1, 21 and 22. The dependent claims are directed to optional and preferred features.

The apparatus and method of the present invention minimizes the complexity associated with manipulating a vascular filter during an angioplasty or stent placement procedure by incorporating the filter on a catheter guide wire such that the guide wire performs the dual functions of guiding the catheter to a stenosed location, and filtering dislodged particles flowing downstream of the treated area. Moreover, because the guide wire operates independently of the catheter, relative spacing between the filter and the lesion location may be easily altered, and exchanges of various devices over the wire are possible.

To realize the advantages described above, the invention, in one form, comprises a vascular filter guide wire for directing precision placement of a catheter or stent proximate a lesion and selectively filtering particulate debris dislodged by treatment. The guide wire includes an actuating mechanism and an elongated flexible core wire having a proximal end mounted to the actuating mechanism and a distal end for insertion through a vasculature to a position downstream of the lesion. A tubular flexible shaft is slidably disposed telescopically along the core wire. The shaft includes a proximal portion affixed to the actuating mechanism in movable relation to the wire proximal end, and a distal portion disposed inwardly from the core wire distal end for placement downstream of the lesion. A collapsible strainer coupled to the shaft distal portion is operable, in response to relative displacement between the shaft and the core wire, to radially extend outwardly within the vasculature so that it can trap particulate matter arising from the treatment of the lesion.

In another form, the invention comprises a catheter system for treating a lesion within the vasculature. The catheter system includes a catheter having a lesion treatment device and a vascular filter guide wire for directing the catheter to the lesion. The guide wire includes a collapsible filter for deployment downstream of the catheter to trap particulate matter dislodged from the lesion during the treatment.

The guide wire of the invention facilitates a method of filtering particulate debris from a vasculature caused by treatment of a lesion with a catheter having a lesion treatment portion, the catheter being guided to the location of the lesion by a vascular filter guide wire having a core wire, a slidable shaft, and a collapsible filter mounted on the shaft and deployable upon relative displacement between the core wire and the shaft. The method includes the steps of first guiding the vascular filter guide wire through the vasculature along a predetermined path to a lesion such that the filter is disposed downstream of the lesion. The next step involves deploying the filter radially outwardly by shifting the shaft relative to the core wire. Then, the catheter is run over the guide wire along the predetermined path to position the lesion treatment portion of the catheter proximate the lesion. The method continues by treating the lesion according to a predetermined procedure then maintaining the filter in a deployed position until the risk of particulate matter is substantially eliminated. The catheter is then withdrawn from the vasculature and the filter retracted radially inwardly by shifting the shaft back to the original position. The method then concludes with the step of removing the guide wire from the vasculature.

One embodiment comprises a vascular filter for controllably expanding within a blood vessel to trap particulate matter.loosened from treatment of a lesion. The filter is responsive to relatively shiftable control elements to expand and retract and includes a braid comprising a composite metallic/polymeric material. The material includes a plurality of metallic filaments mounted to the respective shiftable shaft and core wire to define a support structure and a polymeric mesh interwoven with the metallic filaments to define a strainer.

Another form of the invention comprises a method of fabricating a vascular filter. The method includes the steps of first selecting a mandrel having a plurality of consecutively connected forms and weaving a continuous layer of braid over the consecutively connected forms. The method proceeds by bonding the braid filaments at spaced apart sections between respective forms and separating the respective braided forms at the bonded sections. The forms are then removed from the layer of braid.

Other features and advantages of the present invention will be apparent from the following detailed description when read in conjunction with the accompanying drawings.

### BRIEF DESCRIPTION OF THE DRAWINGS

FIG. 1 is an enlarged, partial sectional view of a catheter system of the present invention deployed within a blood vessel;
FIG. 2 is a partial longitudinal view of a guide wire in a retracted position according to a first embodiment of the present invention;
FIG. 3 is a partial longitudinal sectional view along line 3-3 of FIG. 2;
FIG. 4 is a partial longitudinal sectional view similar to FIG. 3 but in a deployed orientation;
FIG. 5 is an enlarged view of detail 5-5;
FIG. 6 is a longitudinal view of a filter construction according to an alternative embodiment of the present invention;
FIG. 7 is a longitudinal view of a filter construction according to yet another embodiment of the present invention;
FIG. 8 is a longitudinal view of a mandrel system for use in the method of the present invention;
FIG. 9 is a block diagram illustrating steps in preparing the mandrel of FIG. 8;
FIG. 10 is a block diagram illustrating steps in fabricating the filter of the present invention;
FIG. 11a - 11g are views of various stages of construction corresponding to the steps of FIG. 10;
FIG. 12 is a partial longitudinal sectional view of a guide wire in a retracted state according to a second embodiment of the present invention;
FIG. 13 is a partial view of the guide wire of FIG. 12 in an extended state;
FIG. 14 is an axial view along line 14-14 of FIG. 13;
FIG. 15 is an axial view similar to FIG. 14 and showing an alternative strut arrangement; and
FIG. 16 is an axial view similar to FIG. 14 and showing an alternative strut arrangement.

### DETAILED DESCRIPTION OF THE INVENTION

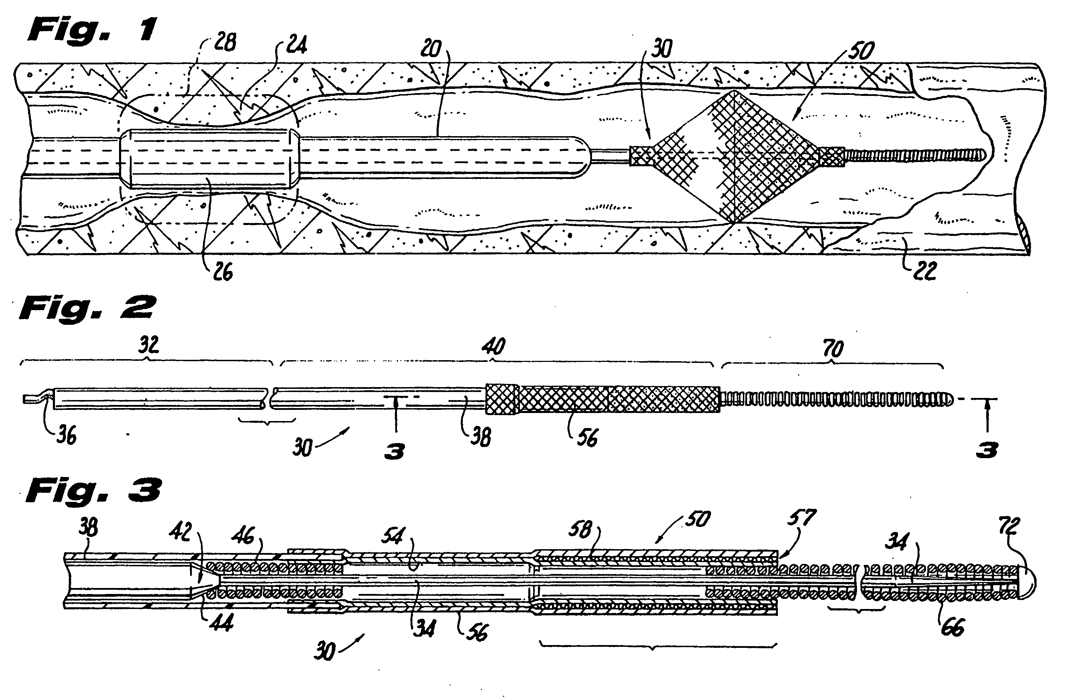

With reference to Figure 1, percutaneous angioplasty or stent placement techniques enable operators to minimize trauma often associated with more invasive surgical techniques. This is possible through the use of a thin catheter 20 that advances through the vascular system to a predetermined blood vessel 22 having a lesion such as a stenosis 24 blocking the flow of blood therethrough. Typically, the catheter includes a lesion treatment device such as a balloon 26 or stent (not shown) for positioning coaxially within the lesion. Once positioned, the balloon or stent radially expands, as shown at 28, to exert a radially outwardly directed force against the material and initiate dilation thereof.

In order to reach the lesioned area, however, the catheter must be able to follow a trackable path defined by a catheter guide wire. In accordance with a first embodiment of the present invention, a catheter guide wire, generally designated 30, provides a trackable path for a catheter and includes a distally disposed collapsible filter 50 to trap particulate matter dislodged by the catheter 20 during treatment of the stenosis.

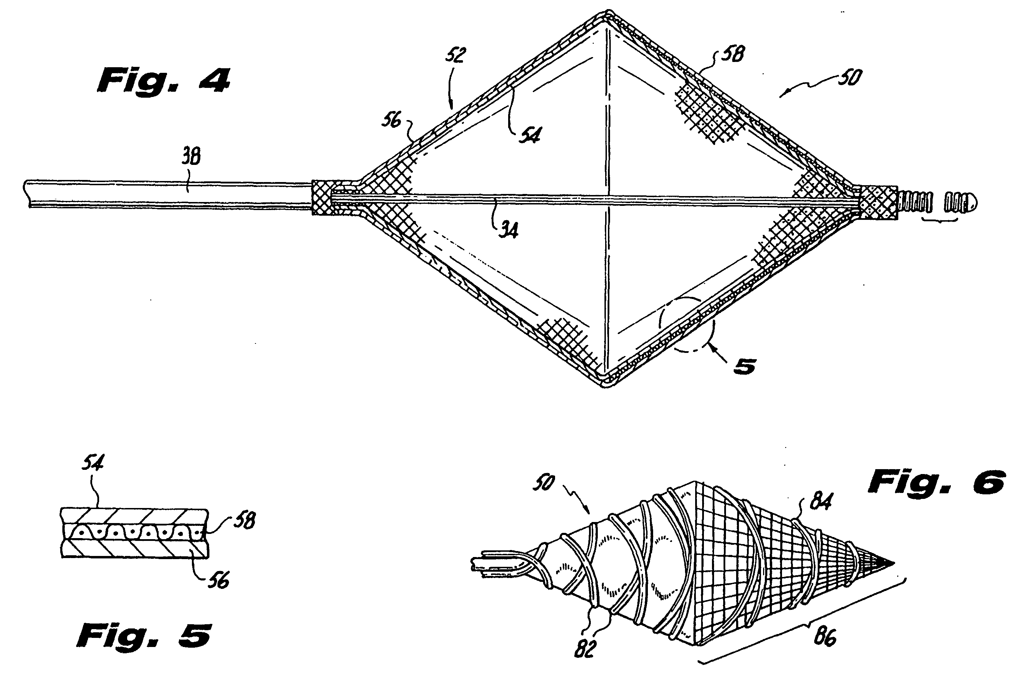

Referring now to Figures 2 through 5, the guide wire 30 includes a proximal section 32 comprising a solid core wire 34 having a wave-shaped proximal end 36 (Figure 2). A tubular shaft 38 is coaxially disposed around the core wire and includes an outer diameter equal to the nominal size of the guide wire. The inner diameter of the tube is sized to form a friction fit with the core wire proximal end when slid thereover during insertion and removal of the guide wire. The shaft functions to deploy and retract the filter device, and to guide and support the catheter 20, and to smoothly transmit rotation from the proximal section 32 to an intermediate section 40. Preferably, the shaft comprises a polyimide tube or hypotube. In some applications, where relatively long lengths are required, an extension (not shown) may be attached to the proximal section to increase the length up to three meters.

The intermediate section 40 extends axially from the proximal section 32 and generally comprises an extension of the shaft 38 to coaxially surround the core wire 34. The core wire is formed distally with a primary tapered portion 42 defining an annular shoulder 44 for mounting a coiled spring 46. with further reference to Figures 2 through 5, the filter 50 comprises a braided basket 52 having respective inner and outer braid layers 54 and 56 (Figure 5) that, in one embodiment, serve as supports for a fine filter mesh 58. The supports expand the basket radially outwardly with the filter axial ends compressed inwardly, and radially retract the basket with the ends tensioned outwardly. The fine mesh 58 (Figure 5) is interposed between the inner and outer supports along a distal-half portion 60 of the basket to prevent particulate matter from flowing through the blood vessel downstream of the treated stenosis. It is contemplated that the size of the pores of mesh 58 may be in the range of 40 to 500 microns. The meshed distal-half of the filter forms a collection cavity 62 for the material such that when retracted, the material is prevented from escaping the filter.

The proximal end of the filter basket is bonded (e.g. adhesively or by soldering) to the shaft 38 which may be inserted between braid layers 54 and 56.

The distal extremity 57 of basket abuts a flexible coil spring 66 that coaxially surrounds the tip of the core wire 34. The guide wire distal tip is tapered and terminates in a hemispherically shaped tip 72 which is also bonded (e.g. by soldering) to the tip. The guide wire distal tip may be pre-formed into a "J" configuration (not shown) to aid in advancing the guide wire 30 through the vasculature.

With particular reference to Figure 6, the preferred embodiment of filter 50 according to the present invention includes a braid comprising a composite metallic/polymeric material, eliminating the necessity of a separate mesh layer. In such an embodiment, a plurality of metallic filaments 82 provide structural support to the assembly for deploying and collapsing the filter. Polymeric filaments 84 are located on the distal half of the braid only, to provide a filtration cone 86. The dual materials, braided simultaneously, provide a pic density which will result in filtration spacing of approximately 40 to 500 microns for filtration, at a metal to polymeric ratio of approximately 1:4.

In yet another embodiment of a filter according to the present invention, generally designated 90 and illustrated in Figure 7, the filtering medium is wrapped in a cylinder 92 with a closed distal end 94 and a flared proximal end 95. Flaring of the proximal end may be effected by applying heat and pressure to the material thereby increasing the surface area and causing the material to bow radially outwardly. The cylinder is formed with longitudinal pleats (not shown) that are more flexible and collapsible than a straight cone configuration.

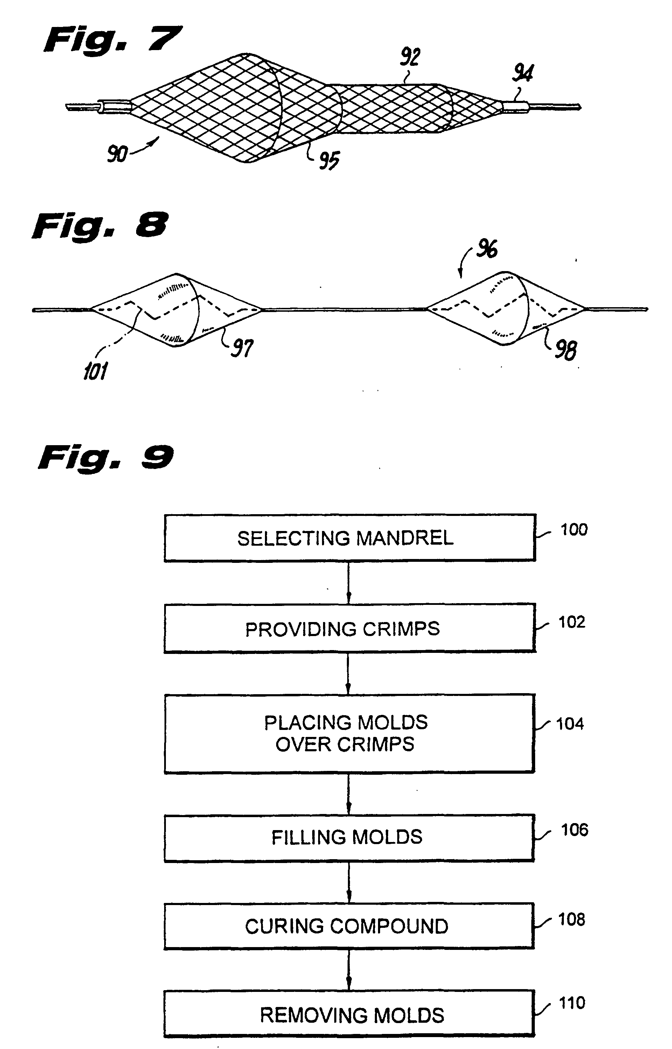

Referring now to Figures 8 and 9, fabrication of the filter 50 may be performed in accordance with a series of process steps as described below. Initially, a mandrel 96 (Figure 8) with a series of molded forms 97 and 98 is prepared by selecting a mandrel of appropriate length, at step 100 (Figure 9), and providing a plurality of crimps 101 (Figure 8) on the mandrel at intervals of approximately two to three inches, at step 102. The process proceeds by placing molds over the crimps, at step 104, filling the molds with a dissolvable compound, at step 106, curing the compound, at step 108, and removing the molds, at step 110. Suitable materials for molding include water soluble plastics such as polyethylene oxide, chemical soluble plastics such as styrene or PVC, and other water soluble materials such as sugar cubes, or gypsum based compounds. Molded forms may be continuously fabricated along the length of the crimped mandrel sections to maximize production efficiency. Another suitable method envisioned is to individually form the molds and bond to straight mandrels.

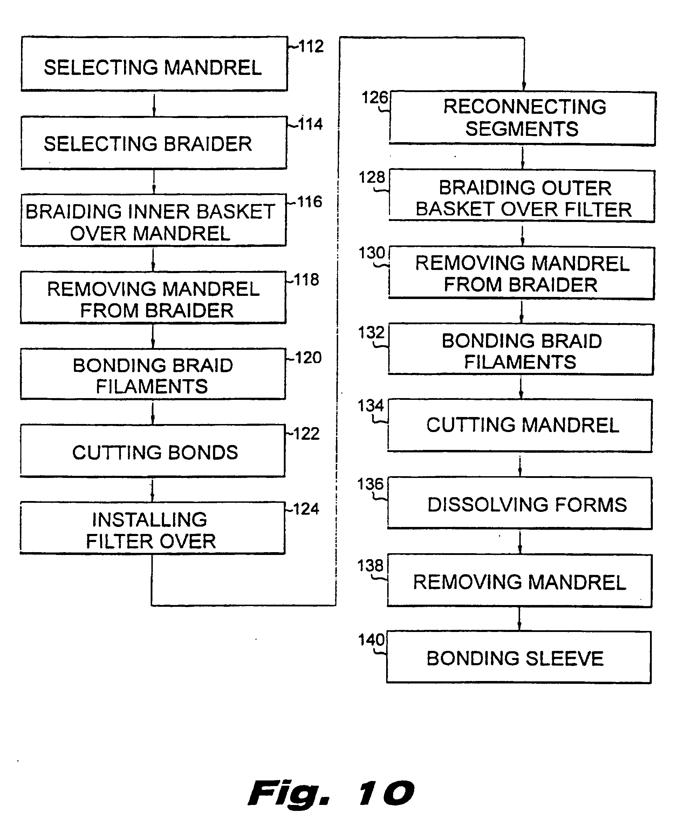

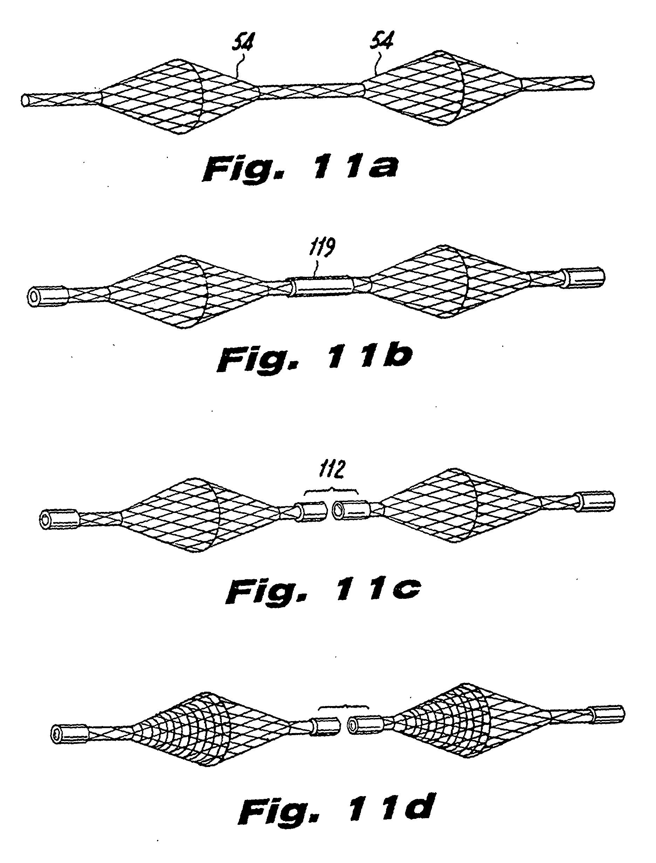

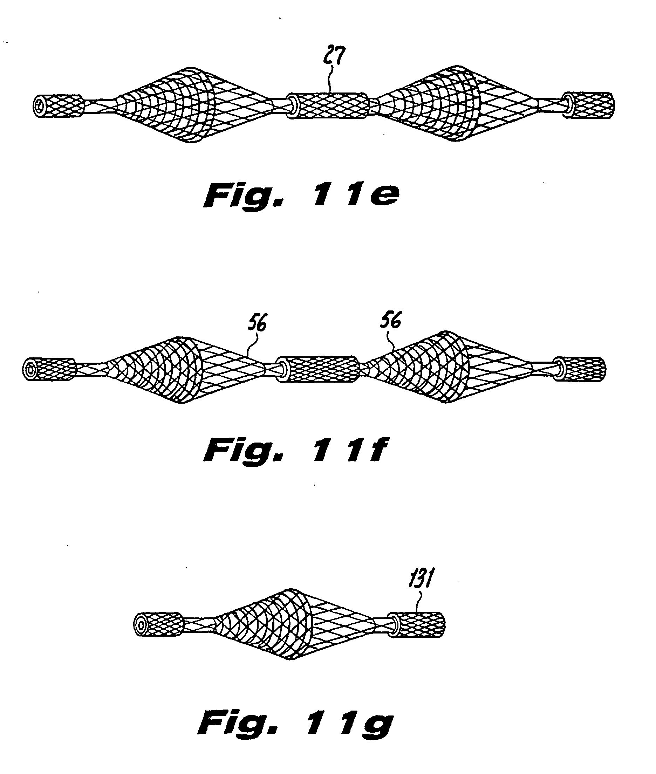

Referring now to Figures 10 and 11a-g, following preparation of the mandrel 96, the mandrel itself is selected for the method of fabricating the filters, at step 112. The method progresses by selecting a braider, at step 114, and braiding the inner layer 54 (Figure 11a), at step 116, over the mandrel form system. Because of the convenient serially connected system of forms on the mandrel, the braider progressively weaves a continuous layer of braid over the consecutively connected forms. After the braid is applied, the mandrel is removed from the braider, at step 118, so that a curable epoxy may be applied to define an adhesive joint 119 (Figure 11b) along spaced apart sections of the braid between forms. This step bonds braid filaments together, at step 120, so that subsequent separation of the forms minimizes deformation of the braid.

A center section 121 (Figure 11c) of each braid is then cut, at step 122, and a prefabricated filter 123 (Figure 11d) installed over one side of each form, at step 124. The individual segments are then reconnected, at step 126, by splicing a section of heat shrink tubing 127 (Figure 11e) over each severed joint.

After the segments are re-connected, the mandrel assembly is then re-installed into the braider for braiding of the outer basket 56 (Figure 11f), at step 128. Following braiding, the mandrel is removed from the braider, at step 130, with the braid filaments bonded together to form a joint 131 (Figure 11g), at step 132. The mandrel is then cut at approximately one millimeter on the outside end of the adhesive, at step 134. At this point, the molded form may be dissolved by an appropriate solvent, at step 136, and the mandrel removed, at step 138. Lastly, a polyimide sleeve is bonded, at step 140, to the end opposite the filter.

The alternative filter embodiment 80 may be fabricated similar to the procedure above with only minor variations. Conveniently, because of the composite nature and relatively high pic density of the metallic/polymeric braid, only one braiding step is required. After the final braid, the polymeric strands at the proximal end are mechanically or thermally cut away, and the filaments fused at the large diameter of the formed cone to form the collection cavity and to allow for greater blood flow.

In operation, the guide wire 30 may be advanced through a vascular system in any conventional manner to establish a path for the catheter to track over. Generally, as shown in Figure 1, the guide wire is inserted through the lesion and disposed downstream of the lesion 24 a variably selected distance. The distance selected by the operator may be conveniently adjusted merely by further advancing or slightly withdrawing the guide wire. This provides the highly desirable capability of enabling the operator to independently adjust the selected distance to preclude the possibility of embolic material progressing through a branch path between the lesion and the filter. The catheter 20 is then inserted along the guide wire to access the treatment area. Typically, image scanning techniques aid in the exact positioning of the catheter relative to the lesion such that the lesion treatment device will have maximum effectiveness.

The filter may then be deployed by actuating an actuating mechanism (not shown) coupled to the core wire 34 for axially moving the shaft 38 relative to the core wire. As the shaft advances axially along the core wire in the distal direction, the filter basket 52, having its distal end 57 attached to the fixed core wire and its proximal end connected to the shaft, compresses axially and expands radially outwardly against the inner walls of the blood vessel. In its expanded state, the filter 50 collects any plaque that may have loosened and become dislodged from the treated area.

Once the treatment concludes, and the catheter is withdrawn from the body, the filter is retracted radially inwardly by shifting the shaft back to its original position. As the filter retracts, the collection cavity 62 traps any material strained against the filter layer. The guide wire itself is then carefully withdrawn from the vasculature.

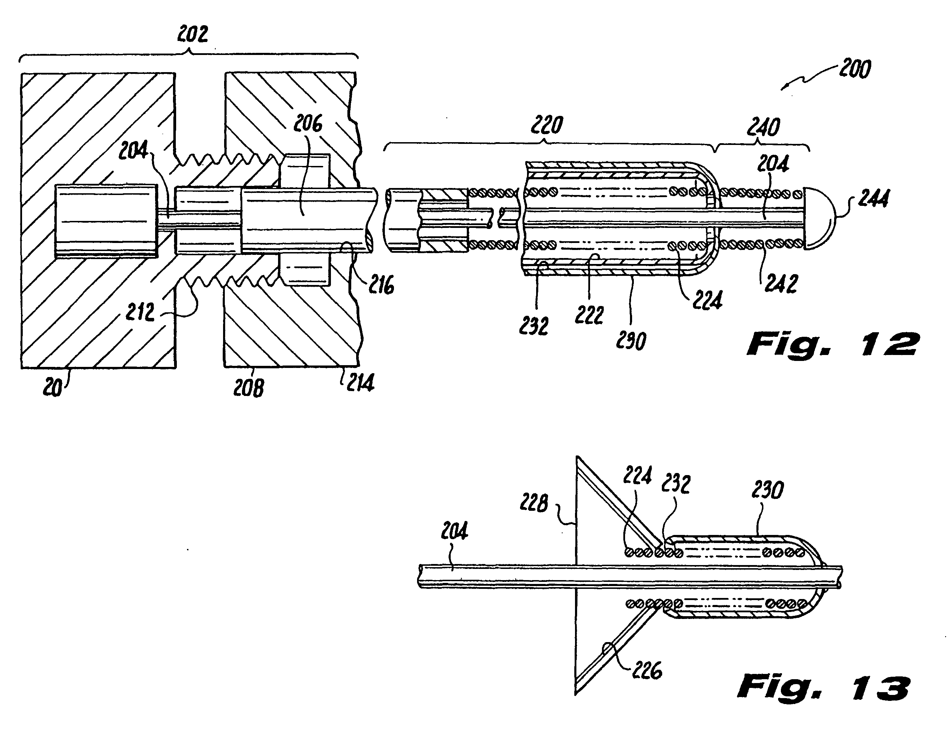

Referring now to Figures 12 through 16, a temporary filter guide wire according to a further embodiment of the present invention is shown, and generally designated 200. The guide wire generally includes a proximal end 202 having an actuating mechanism 208, an intermediate portion 220 including a housed collapsible filter element 222, and a flexible distal end 240.

With particular reference to Figure 12, the proximal end 202 includes a solid stainless steel core wire 204 having a diameter, for example, of approximately 0.1905 mm (approximately 0.0075 inches) and slidably confined coaxially by an elongated shaft 206. The shaft may include, for example, an inner diameter of approximately 0.254 mm (approximately 0.010 inches) and an outer diameter of approximately 0.3556 mm (approximately 0.014 inches). The proximal tip of the core wire nests within the handle mechanism 208 that includes a rotatable handle element 209 having a formed central blind bore 210 and a threaded hollow shank 212. A fixed threaded base 214 having a throughbore 216 receives the proximal portion of the shaft 206 and rotatably engages the handle element to define the actuating mechanism.

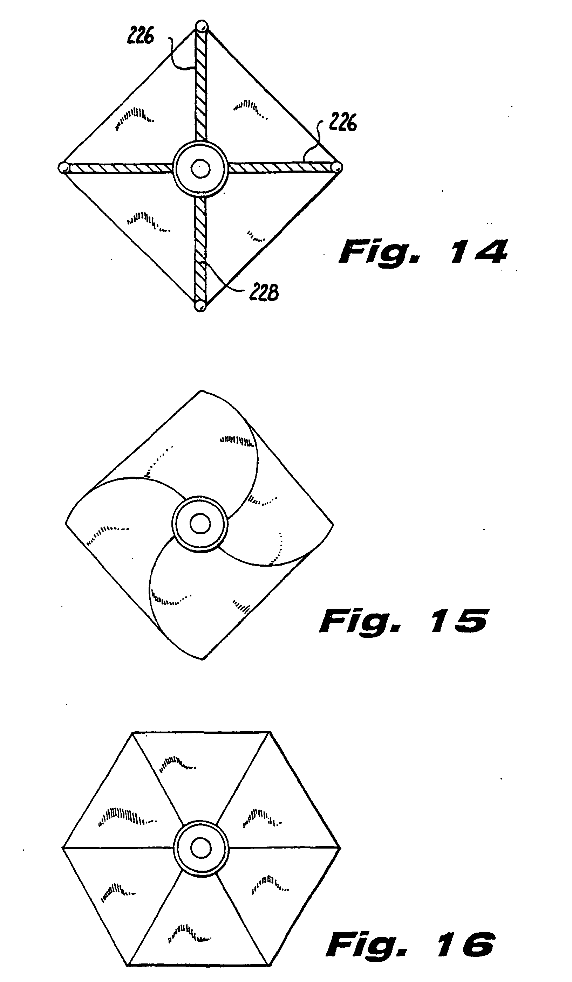

Referring now to Figures 12 and 13, the core wire 204 and the shaft 206 extend longitudinally to define the intermediate portion 220 of the guide wire. The filter element 222 is mounted to the intermediate portion and includes an intermediate quad filar spring 224 of approximately 0.0508 mm (approximately 0.002 inch) diameter wire that extends approximately three to seven centimeters from the end of the shaft, depending on the application. The respective ends of four wires comprising the quad spring are unwound, straightened, and outwardly biased approximately forty-five degrees from the spring axis at spaced apart radial locations to define a plurality of umbrella shaped filter struts 226. These struts form the support structure for the filter. As shown in Figures 14, 15, and 16, the strut spacing may conveniently take on a variety of configurations depending on the particular application desired. Lashed to the struts is a fine wire mesh 228 of approximately 0.0254 mm (approximately 0.001 inches) thick within 40 to 500 micron pores for straining particulate matter from the bloodstream.

Further referring to Figure 12, the radial exterior of the distal portion of the core wire 204 carries a bonded housing or pod 230 having an axially open mouth 232 slightly larger in diameter than the diameter of the filter in a closed configuration. The mouth opens into a cavity sufficiently sized to fully enclose the filter during insertion or withdrawal of the guide wire. The pod would also have a rounded inward edge at its proximal opening so as to envelop the filter when retracted and prevent unintentional engagement of a stent or catheter upon withdrawal. The pod may be fabricated out of a spring material wound in the opposing direction as the spiral struts to improve the sliding of the two surfaces. Other options include a lubricious plastic such as polyethylene.

The distal end 240 of the guide wire 200 comprises an extension of the core wire 204 from a bonded distal joint and surrounded by a distal spring member 242 that bonds to and projects outwardly from the distal side of the filter housing 230. The distal end terminates in a tip 244 that typically takes on a pre-formed "J" shape (not shown) for steering purposes through the vascular system.

Operation of the second embodiment proceeds in much the same way as that of the first embodiment, with the guide wire 200 first directed through the vasculature, followed by tracking with a treating catheter. Like the first embodiment, the guide wire 200 is advantageously adjustable in the blood vessel independent of the catheter, allowing a variable selected distance between the location of the stenosis and the filter. However, the way in which the filter 222 expands and retracts differs somewhat from the previously described embodiment.

With the handle mechanism 208 in a normally open configuration, the operator turns the rotatable element 209 to incrementally drive the core wire 104 axially with respect to the shaft 206. The relative axial displacement of the core wire causes the filter housing 230 to become disengaged from the filter struts 226. Because of the spring biased nature of the filter struts 226, as the filter exits the housing, the struts expand radially outwardly against the blood vessel wall such that the wire mesh spans the vessel diameter. In its extended state, the filter allows bloodflow to continue through the vessel while dislodged material becomes entrapped in the wire mesh for collection in the cavity.

Once the lesion treatment procedure is complete, and the necessity for filtering has completely diminished, the handle mechanism is actuated to pull the core wire back to its original position. This activity causes the housing mouth to re-engage the filter struts and urge the struts radially inwardly as the housing encloses the filter. With the filter fully retracted, the streamlined guide wire may be easily and safely withdrawn from the body.

Those skilled in the art will appreciate the many benefits and advantages afforded the present invention. Of relative importance is the feature that avoids any additional control wires, beyond the guide wire itself, in order to expand and retract the filter. Not only does this minimize the number of components necessary to practice the invention, but the angioplasty procedure itself is made safer for the patient.

Additionally, the present invention provides the capability of adjusting the distance between the filter and the catheter lesion treatment device in vivo, eliminating the need to withdraw the guide wire or catheter for distance adjustment should the relative spacing be inadequate.

The filter itself, in one embodiment, provides substantial manufacturability benefits by requiring only a single braiding step. Consequently, braiding additional filter layers adding to the device's complexity are eliminated. By minimizing the process steps required to fabricate the filter, costs involved in manufacture are greatly reduced.

Moreover, the method of fabricating filters according to the present invention offers added efficiencies in manufacture due to the production line processing scheme. Employing such a scheme serves to dramatically improve the throughput rate of filters to minimize overall costs.

While the invention has been particularly shown and described with reference to the preferred embodiments thereof, it will be understood by those skilled in the art that various changes in form and detail may be made therein without departing from the scope of the invention.

For example, the invention may be used in any intravascular treatment utilizing a guide wire where the possibility of loosening emboli may occur. Although the description herein illustrates angioplasty and stent placement procedures as significant applications, it should be understood that the present invention is in no way limited only to those environments.

## Claims

1. A vascular filter guide wire (30) for directing placement of a catheter (20) with respect to a blood vessel (22) lesion and filtering particulate matter dislodged by treatment of said vessel, said guide wire including:
an elongated flexible core wire (34) having a proximal end (36) and a distal end for insertion and steerage through a patient's vasculature to a position downstream of said lesion;
a tubular flexible shaft (38) slidably disposed along said core wire, said shaft including a proximal portion and a distal portion disposed proximally of said core wire distal end for placement downstream of said lesion; and
a collapsible filter (50) coupled at its proximal end to said distal portion of said shaft, **characterized in that** said collapsible filter (50) is coupled at its distal end to said core wire, wherein said filter is operable in response to the relative displacement between said shaft and said core wire to radially extend outwardly within said vasculature and trap particulate matter arising from the treatment of said lesion;
and wherein said filter is formed with oppositely disposed cone-shaped ends to define proximal (95) and distal (60) portions of the filter and wherein said filter includes a fine filter mesh (58) to make of the distal portion of the filter a collection cavity (62).

2. A vascular filter guide wire according to claim 1 and further including:
a locking mechanism to maintain said filter in a deployed position for particulate filtration during lesion treatment.

3. A vascular filter guide wire according to claim 1 wherein:
said core wire includes a filter housing (230) for confining said filter in a closed configuration.

4. A vascular filter guide according to claim 3 wherein:
said housing is formed in a frusto-conical configuration and axially disposed on said core wire, said housing including an oversized-in-diameter mouth (232) opening axially outwardly from said wire distal end, and a reduced-in-diameter collar radially fixed to said core wire proximate said distal end.

5. A vascular filter guide wire according to claim 3 wherein said strainer is formed to collapsibly engage said housing in a closed state and including:
a plurality of radially spaced apart support struts (226) defining a cage, said struts collapsibly hinged at one end along a common radial path on said shaft and interconnected through a woven peripheral mesh (228); and
a biasing element (224) interposed concentrically between said struts and said shaft to bias said struts radially outwardly in an open state.

6. A vascular filter guide wire according to claim 5 wherein:
said spaced apart struts are disposed radially equidistant.

7. A vascular filter guide wire according to claim 5 wherein:
said spaced apart struts are disposed in a spiral relationship.

8. A vascular filter guide wire according to claim 5 wherein:
said struts are formed of a high elastic material.

9. A vascular filter guide wire according to claim 5 wherein:
said woven mesh comprises a polymeric material.

10. A vascular filter guide wire according to claim 5 wherein:
said woven mesh density is in the range 40 to 500 micrometers.

11. A vascular filter guide wire according to claim 5 wherein:
said biasing element comprises a quad filar spring.

12. A vascular filter guide wire according to claim 1, further including a deployment/retraction mechanism including:
a removable base (214) formed with a threaded passage for confining the proximal portion of said shaft; and
a manually rotatable control element (209), said control element formed with a threaded hollow shank (212) and mounted to the proximal end of said wire, said control element operable to threadably engage said passage and incrementally urge relative axial displacement between said shaft and said wire to extend and retract said filter.

13. A vascular filter guide wire according to claim 1 wherein said filter includes:
a cylindrical support cage (92) having a closed distal end (94) and a flared proximal end (95), said distal end fixed to the distal extremity of said shaft, and said proximal end extending axially and mounted to said core wire distal end; and
a continuous woven mesh having a plurality of longitudinal pleats and disposed within said support cage for straining particulate matter.

14. A vascular filter guide wire according to claim 13 wherein
said woven mesh is mounted to back half of said cage.

15. A vascular filter guide wire according to claim 13 wherein:
said woven mesh comprises a material from the group including stainless steel and nickel-titanium alloy.

16. A vascular filter guide wire according to claim 13 wherein:
said wire mesh density is in the range 40 to 500 micrometers.

17. A vascular filter guide wire according to claim 1 wherein said filter includes:
a braid comprising a composite metallic/polymeric material, said material including
a plurality of metallic filaments (82) mounted to said respective shiftable shaft and core wire to define a support structure and
as the fine filter mesh a polymeric mesh interwoven with said metallic filaments to define a strainer.

18. A vascular filter guide wire according to claim 17 wherein:
said metallic filaments have respective common proximal and distal halves; and
said polymeric mesh is interwoven in said distal half of said metallic filaments.

19. A vascular filter guide wire according to claim 18 wherein:
said metallic and polymeric filaments are woven at a ratio of approximately 1:4.

20. A guide wire as claimed in any one of the preceding claims, including
an actuating mechanism and wherein the proximal end of the elongated flexible core wire is attached to said actuating mechanism position downstream of said lesion; and wherein the proximal end of the tubular flexible shaft is affixed to said actuating mechanism.

21. A catheter system for treating a blood vessel lesion within a vasculature, said catheter system including:
a catheter having a lesion treatment device (26); and
a vascular filter guide wire as claimed in any one of the preceding claims.

22. A method of fabricating a vascular filter guide wire as claimed in any one of claims 1 to 20, said method including the steps of:
selecting a mandrel (96) having a plurality of consecutively connected forms (97, 98);
weaving a continuous layer of braid over said consecutively connected forms;
bonding said braid filaments at spaces apart sections between respective forms;
separating said respective braided forms at said bonded sections;
removing said forms from said layer of braid to create said collapsible filter; and coupling said collapsible filter at its proximal end to said distal portion of said shaft and at its distal end to said core wire.

23. A method of fabricating a vascular filter guide wire according to claim 22, wherein said step of weaving includes:
forming a layer of braid over the proximal and distal halves of each form with a composite metallic/polymeric material to having a pic density sufficient to strain particulate matter.

24. A method of fabricating a vascular filter guide wire according to claim 23 and further including the step of:
cutting said polymeric filaments from said proximal halves of each form; and
fusing the ends of said cut filaments to form a collection cavity (62) around each form.

25. A method of fabricating a vascular filter guide wire according to claim 22 wherein after said bonding step, said method further includes the steps of:
installing a filter layer over each form;
weaving a second continuous layer of braid having a plurality of second braid filaments over said installed filters; and
bonding said second braid filaments at said spaced apart sections.

26. A method of fabricating a vascular filter guide wire according to claim 22, wherein said forms are molded from a dissolvable material, said step of removing including:
dissolving said forms by an appropriate solvent.

## Patentansprüche

1. Gefäßfilter-Leitdraht (30), um die Plazierung eines Katheters (20) bezüglich einer Blutgefäß-(22)Läsion zu lenken, und durch die Behandlung des Blutgefäßes entfernte Feststoffe zu filtern, wobei der Leitdraht aufweist:
einen länglichen flexiblen Kerndraht (34) mit einem proximalen Ende (36) und einem distalen Ende zur Einführung und Steuerung durch die Vaskulatur eines Patienten an eine Position stromabwärts der Läsion;
einen rohrförmigen flexiblen Schaft (38), der gleitbar entlang dem Kerndraht angeordnet ist, wobei der Schaft einen proximalen Abschnitt und einen distalen Abschnitt, der nahe dem distalen Ende des Kerndrahts zur Plazierung stromabwärts der Läsion angeordnet ist, aufweist; und
einen zusammenlegbaren Filter (50), der an seinem distalen Ende mit dem distalen Abschnitt des Schafts verbunden ist, **dadurch gekennzeichnet, dass** der zusammenlegbare Filter (50) an seinem distalen Ende mit dem Kerndraht verbunden ist, wobei der Filter als Antwort auf die relative Verschiebung zwischen dem Schaft und dem Kerndraht betriebsbereit ist, sich innerhalb der Vaskulatur radial nach außen zu erstrecken und Feststoffe, die auf die Behandlung der Läsion zurückzuführen sind, einzuschließen;
und wobei der Filter mit gegenüberliegend angeordneten kegelförmigen Enden gebildet ist, um proximale (95) und
distale (60) Abschnitte des Filters zu begrenzen, und wobei der Filter ein feines Filtergewebe (58) aufweist, um aus dem distalen Abschnitt des Filters einen Sammlungsraum (62) zu machen.

2. Gefäßfilter-Leitdraht nach Anspruch 1 und ferner mit einem Arretiermechanismus, um den Filter in einer entfalteten Stellung zur Filtration von Feststoffen während einer Läsionsbehandlung zu halten.

3. Gefäßfilter-Leitdraht nach Anspruch 1, bei dem der Kerndraht ein Filtergehäuse (230) zur Begrenzung des Filters in einer geschlossenen Konfiguration aufweist.

4. Gefäßfilter-Leitdraht nach Anspruch 3, bei dem das Gehäuse in einer stumpfkegeligen Konfiguration gebildet und axial auf dem Kerndraht angeordnet ist, wobei das Gehäuse eine im Durchmesser übergroße Öffnung (232), die sich axial nach außen von dem distalen Ende des Drahts öffnet, und einen im Durchmesser reduzierten Bund, der radial an dem Kerndraht nahe dem distalen Ende befestigt ist, aufweist.

5. Gefäßfilter-Leitdraht nach Anspruch 3, bei dem der Filter (strainer) geformt ist, um in einem geschlossenen Zustand mit dem Gehäuse zusammenlegbar in Eingriff zu sein und mit:
einer Vielzahl von radial beabstandeten Stützstreben (226), die einen Käfig begrenzen, wobei die Streben an einem Ende entlang eines gemeinsamen radialen Wegs auf dem Schaft zusammenlegbar gelenkig verbunden sind und durch ein gewebtes Umfangsgewebe (228) miteinander verbunden sind; und
einem Vorspannelement (224), das konzentrisch zwischen den Streben und dem Schaft angeordnet ist, um die Streben radial nach außen in einen geöffneten Zustand vorzuspannen.

6. Gefäßfilter-Leitdraht nach Anspruch 5, bei dem die beabstandeten Streben radial mit gleichem Abstand angeordnet sein.

7. Gefäßfilter-Leitdraht nach Anspruch 5, bei dem die beabstandeten Streben in einer spiralförmigen Verbindung angeordnet sind.

8. Gefäßfilter-Leitdraht nach Anspruch 5, bei dem die beabstandeten Streben aus einem hochelastischen Material gebildet sind.

9. Gefäßfilter-Leitdraht nach Anspruch 5, bei dem das gewebte Gewebe ein polymerisches Material aufweist.

10. Gefäßfilter-Leitdraht nach Anspruch 5, bei dem die Dichte des gewebten Gewebes in dem Bereich von 40 bis 500 Mikrometer liegt.

11. Gefäßfilter-Leitdraht nach Anspruch 5, bei dem das Vorspannelement eine Vierfadenfeder (quad filar spring) aufweist.

12. Gefäßfilter-Leitdraht nach Anspruch 1, ferner mit einem Entfalten/Einziehen-Mechanismus mit:
einer entfernbaren Basis (214), die mit einem Durchgang mit Gewinde zur Begrenzung des proximalen Abschnitts des Schafts gebildet ist; und
einem manuell drehbaren Steuerelement (209), wobei das Steuerelement mit einem hohlen Schaft (212) mit Gewinde gebildet und an dem proximalen Ende des Drahts angebracht ist, wobei das Steuerelement betriebsfähig ist, mittels des Gewindes in den Durchgang einzugreifen und inkrementell eine relativ axiale Verschiebung zwischen dem Schaft und dem Draht voranzutreiben, um den Filter auszufahren oder einzuziehen.

13. Gefäßfilter-Leitdraht nach Anspruch 1, bei dem der Filter aufweist:
einen zylindrischen Stützkäfig (92) mit einem geschlossenen distalen Ende (94) und einem konisch erweiterten proximalen Ende (95), wobei das distale Ende an dem distalen Ende des Schafts befestigt ist, und sich das proximale Ende axial erstreckt und an dem distalen Ende des Kerndrahts angebracht ist; und
einem kontinuierlich gewebten Gewebe, das eine Vielzahl von länglichen Falten aufweist und innerhalb des Stützkäfigs angeordnet ist, um Feststoffe zu filtern.

14. Gefäßfilter-Leitdraht nach Anspruch 13, bei dem das gewebte Gewebe an der rückseitigen Hälfte des Käfigs angebracht ist.

15. Gefäßfilter-Leitdraht nach Anspruch 13, bei dem das gewebte Gewebe ein Material aus der Gruppe, die rostfreien Stahl und Nickel-Titan-Legierung einschliesst, aufweist.

16. Gefäßfilter-Leitdraht nach Anspruch 13, bei dem die Dichte des gewebten Gewebes in dem Bereich von 40 bis 500 Mikrometer liegt.

17. Gefäßfilter-Leitdraht nach Anspruch 1, bei dem der Filter aufweist:
ein Geflecht mit einem metallischen/polymerischen Verbundmaterial, wobei das Material aufweist:
eine Vielzahl von metallischen Fasern (82), die an den jeweils verschiebbaren Schaft und Kerndraht angebracht sind, um eine Stützstruktur zu begrenzen und
als das feine Filtergewebe ein polymerisches Gewebe, bei dem die metallischen Fäden eingewebt sind, um einen Filter zu begrenzen.

18. Gefäßfilter-Leitdraht nach Anspruch 17, bei dem die metallischen Fäden jeweils gemeinsame proximale und distale Hälften aufweisen; und
das polymerische Gewebe in die distale Hälfte der metallischen Fäden eingewebt ist.

19. Gefäßfilter-Leitdraht nach Anspruch 18, bei dem die metallischen und polymerischen Fasern in einem Verhältnis von ungefähr 1:4 gewebt sind.

20. Leitdraht nach einem der vorstehenden Ansprüche, mit einem Betätigungsmechanismus und wobei das proximale Ende des länglichen flexiblen Kerndrahts an dem Betätigungsmechanismus, der stromabwärts von der Läsion positioniert ist, angebracht ist; und
wobei das proximale Ende des rohrförmigen flexiblen Schafts an dem Betätigungsmechanismus befestigt ist.

21. Katheter-System zur Behandlung einer Blutgefäßläsion innerhalb einer Vaskulatur, wobei das Katheter-System aufweist:
einen Katheter mit einer Läsionsbehandlungseinrichtung (26); und
einen Gefäßfilter-Leitdraht nach einem der vorstehenden Ansprüche.

22. Verfahren zur Herstellung eines Gefäßfilter-Leitdrahts nach einem der Ansprüche 1 bis 20, wobei das Verfahren die folgenden Schritte aufweist:
Wählen eines Dorns (96) mit einer Vielzahl von aufeinanderfolgenden verbundenen Formen (97, 98);
Weben einer kontinuierlichen Geflechtschicht über die aufeinanderfolgenden verbundenen Formen;
Verbinden der Geflechtfasern an von einander beabstandeten Abschnitten zwischen den jeweiligen Formen;
Trennen der jeweiligen geflochtenen Formen an den verbundenen Abschnitten;
Entfernen der Formen von der Geflechtschicht, um den zusammenlegbaren Filter zu erzeugen; und
Koppeln des zusammenlegbaren Filters an seinem proximalen Ende mit dem distalen Abschnitt des Schafts und an seinem distalen Ende mit dem Kerndraht.

23. Verfahren zur Herstellung eines Gefäßfilter-Leitdrahts nach Anspruch 22, wobei der Schritt des Webens aufweist:
Bilden einer Geflechtschicht über der proximalen und der distalen Hälfte von jeder Form mit einem metallischen/polymerischen Verbundmaterial um eine Bilddichte (pic density) aufzuweisen, die ausreichend ist, um Feststoffe zu filtern.

24. Verfahren zur Herstellung eines Gefäßfilter-Leitdrahts nach Anspruch 23, und ferner den folgenden Schritt aufweisend:
Schneiden der polymerischen Fasern von den proximalen Hälften von jeder Form; und
Schmelzen der Enden der geschnittenen Fasern um einen Sammlungsraum (62) um jede Form zu bilden.

25. Verfahren zur Herstellung eines Gefäßfilter-Leitdrahts nach Anspruch 22, bei dem nach dem Verbindungsschritt das Verfahren ferner die folgenden Schritte aufweist:
Anbringen einer Filterschicht über jeder Form;
Weben einer zweiten kontinuierlichen Geflechtschicht mit einer Vielzahl von zweiten Geflechtfasern über den angebrachten Filtern; und
Verbinden der zweiten Geflechtfasern an den beabstandeten Abschnitten.

26. Verfahren zur Herstellung eines Gefäßfilter-Leitdrahts nach Anspruch 22, bei dem die Formen aus einem lösbaren Material gegossen sind, wobei der Schritt des Entfernens aufweist:
Auflösen der Formen durch ein geeignetes Lösungsmittel.

## Revendications

1. Fil de guidage (30) de filtre vasculaire pour guider le positionnement d'un cathéter (20) par rapport à une lésion d'un vaisseau sanguin (22) et filtrer une matière particulaire détachée sous l'effet du traitement dudit vaisseau, ledit fil de guidage comprenant:
un fil formant noyau flexible allongé (34) possédant une extrémité proximale (36) et une extrémité distale pour l'insertion et la conduite au sein de la vasculature d'un patient vers une position située en aval de ladite lésion;
un conduit tubulaire flexible (38) disposé de manière à pouvoir glisser le long dudit fil formant noyau, ledit conduit comprenant une partie proximale et une partie distale disposée sur le côté proximal de ladite extrémité distale du fil formant noyau pour un positionnement en aval de ladite lésion; et
un filtre repliable (50) couplé, au niveau de son extrémité proximale, à ladite partie distale dudit conduit;
**caractérisé en ce que** ledit filtre repriable (50) est couplé, au niveau de son extrémité distale, audit fil formant noyau, et dans lequel ledit filtre peut être mis en service en réponse au déplacement relatif entre ledit conduit et ledit fil formant noyau pour s'étendre radialement vers l'extérieur dans ledit système vasculaire et piéger une matière particulaire apparaissant sous l'effet du traitement de ladite lésion;
et dans lequel ledit filtre est formé avec des extrémités de forme conique disposées dans des positions opposées afin de définir une partie proximale (95) et une partie distale (60) du filtre, et dans lequel ledit filtre inclut une structure maillée fine de filtre (58) de manière que la partie distale du filtre forme une cavité de collecte (62).

2. Fil de guidage de filtre vasculaire selon la revendication 1 et comprenant en outre:
un mécanisme de blocage pour maintenir ledit filtre dans une position déployée pour la filtration de particules pendant le traitement d'une lésion.

3. Fil de guidage de filtre vasculaire selon la revendication 1, dans lequel:
ledit fil formant noyau comprend un boîtier de filtre (230) pour confiner ledit filtre dans une configuration fermée.

4. Fil de guidage de filtre vasculaire selon la revendication 3, dans lequel:
ledit boîtier est agencé avec une configuration tronconique et est disposé axialement sur ledit fil formant noyau, ledit boîtier incluant une embouchure de diamètre surdimensionné (232) s'ouvrant axialement vers l'extérieur à partir de ladite extrémité distale du fil, et un collet de diamètre réduit fixé radialement audit fil formant noyau à proximité de ladite extrémité distale.

5. Fil de guidage de filtre vasculaire selon la revendication 3, dans lequel ladite crépine est formée de manière à s'appliquer, tout en pouvant se replier, sur ledit boîtier dans un état fermé, et comprenant:
une pluralité d'entretoises de support (226) distantes radialement et définissant une cage, lesdites entretoises étant articulées avec possibilité de se replier, au niveau d'une extrémité, le long d'un trajet radial commun sur ledit conduit, et interconnectées au moyen d'une structure maillée périphérique tissée (228); et
un élément de sollicitation (224) intercalé concentriquement entre lesdites entretoises et ledit conduit de manière à solliciter lesdites entretoises radialement vers l'extérieur dans un état ouvert.

6. Fil de guidage de filtre vasculaire selon la revendication 5, dans lequel:
lesdites entretoises espacées sont disposées en étant radialement équidistantes.

7. Fil de guidage de filtre vasculaire selon la revendication 5, dans lequel:
lesdites entretoises espacées sont disposées selon une relation hélicoïdale.

8. Fil de guidage de filtre vasculaire selon la revendication 5, dans lequel:
lesdites entretoises sont formées d'un matériau très élastique.

9. Fil de guidage de filtre vasculaire selon la revendication 5, dans lequel:
ladite structure maillée tissée comprend un matériau polymère.

10. Fil de guidage de filtre vasculaire selon la revendication 5, dans lequel:
ladite densité de la structure maillée tissée se situe dans la gamme de 40 à 500 micromètres.

11. Fil de guidage de filtre vasculaire selon la revendication 5, dans lequel:
ledit élément de sollicitation comprend un ressort à quatre fils.

12. Fil de guidage de filtre vasculaire selon la revendication 1, comprenant en outre un mécanisme de déploiement / rétraction comprenant:
une base amovible (214) formée d'un passage taraudé pour confiner la partie proximale dudit conduit; et
un élément de commande (209) que l'on peut faire tourner manuellement, ledit élément de commande étant formé d'une tige creuse filetée (212) et étant monté sur l'extrémité proximale dudit fil, ledit élément de commande pouvant agir de manière à mettre en prise par vissage avec ledit passage et appliquer une sollicitation incrémentale avec déplacement axial relatif entre ledit conduit et ledit fil pour étendre et rétracter ledit filtre.

13. Fil de guidage de filtre vasculaire selon la revendication 1, dans lequel ledit filtre comprend:
une cage de support cylindrique (92) possédant une extrémité distale fermée (94) et une extrémité proximale évasée (95), ladite extrémité distale étant fixée à l'extrémité distale dudit conduit, et ladite extrémité proximale s'étendant axialement et étant montée sur ladite extrémité distale du fil formant noyau; et
une structure maillée tissée continue comportant une pluralité de plis longitudinaux et disposée dans ladite cage de support de manière à filtrer une matière particulaire.

14. Fil de guidage de filtre vasculaire selon la revendication 13, dans lequel
ladite structure maillée tissée est montée sur la moitié arrière de ladite cage.

15. Fil de guidage de filtre vasculaire selon la revendication 13, dans lequel:
ladite structure maillée tissée comprend un matériau provenant du groupe comprenant l'acier inoxydable et l'alliage de nickel-titane.

16. Fil de guidage de filtre vasculaire selon la revendication 13, dans lequel:
ladite densité de structure maillée formée d'un fil se situe dans la gamme de 40 à 500 micromètres.

17. Fil de guidage de filtre vasculaire selon la revendication 1, dans lequel ledit filtre comprend:
une tresse comprenant un matériau métallique / polymère composite, ledit matériau incluant
une pluralité de filaments métalliques (82) montés sur ledit conduit et ledit fil formant noyau, respectifs déplaçables pour définir une structure de support; et
en tant que structure maillée fine de filtre, une structure maillée polymère entrelacée avec ledit filament métallique pour définir une crépine.

18. Fil de guidage de filtre vasculaire selon la revendication 17, dans lequel:
lesdits filaments métalliques possèdent des moitiés proximale et distale communes respectives; et
ladite structure maillée polymère est entrelacée avec ladite moitié distale desdits filaments métalliques.

19. Fil de guidage de filtre vasculaire selon la revendication 18, dans lequel:
lesdits filaments métalliques et polymères sont tissés avec un rapport égal à environ 1:4.

20. Fil de guidage selon l'une quelconque des revendications précédentes, incluant:
un mécanisme d'actionnement, et dans lequel l'extrémité proximale du fil formant noyau flexible allongé est fixée à ladite position du mécanisme d'actionnement en aval de ladite lésion; et dans lequel l'extrémité proximale du conduit tubulaire flexible est fixée audit mécanisme d'actionnement.

21. Système de cathéter pour le traitement d'une lésion d'un vaisseau sanguin dans une vasculature, ledit système de cathéter comprenant:
un cathéter possédant un dispositif (26) de traitement de lésion; et
un fil de guidage de filtre vasculaire tel que revendiqué dans l'une quelconque des revendications précédentes.

22. Procédé de fabrication d'un fil de guidage de filtre vasculaire selon l'une quelconque des revendications 1 à 20, ledit procédé incluant les étapes consistant à:
choisir un mandrin (96) possédant une pluralité de formes (97, 98) raccordées successivement;
tisser une couche continue d'une tresse sur lesdites formes raccordées successivement;
réunir lesdits filaments de la tresse dans des sections espacées situées entre des formes respectives;
séparer lesdites formes tressées respectives au niveau desdites sections réunies;
retirer lesdites formes de ladite couche de tresse pour créer ledit filtre repliable; et coupler ledit filtre repliable au niveau de son extrémité proximale, à ladite partie distale dudit conduit et, au niveau de son extrémité distale, audit fil formant noyau.

23. Procédé de fabrication d'un fil de guidage de filtre vasculaire selon la revendication 22, selon lequel ladite étape de tissage inclut:
la formation d'une couche de tresse sur les moitiés proximale et distale de chaque forme avec un matériau métallique / polymère composite de manière à posséder une densité maximale suffisante pour filtrer une matière particulaire.

24. Procédé pour fabriquer un fil de guidage de filtre vasculaire selon la revendication 23, et comprenant en outre l'étape consistant à:
sectionner lesdits filaments polymères desdites moitiés proximales de chaque forme; et
faire fondre les extrémités desdits filaments découpés pour former une cavité de collecte (62) autour de chaque forme.

25. Procédé de fabrication d'un fil de guidage de filtre vasculaire selon la revendication 22, selon lequel après ladite étape de liaison, ledit procédé inclut en outre les étapes consistant à:
installer une couche formant filtre sur chaque forme;
tisser une seconde couche continue de tresse comportant une pluralité de seconds filaments de tresse sur lesdits filtres installés; et
relier lesdits seconds filaments de tresse au niveau desdites sections espacées.

26. Procédé pour fabriquer un fil de guidage de filtre vasculaire selon la revendication 22, selon lequel lesdites formes sont formées par moulage d'un matériau pouvant être dissous, ladite étape d'enlèvement consistant à:
dissoudre lesdites formes à l'aide d'un solvant approprié.
